# EUROPÄISCHE PATENTSCHRIFT

(11) **EP 3 359 102 B1**
(45) Veröffentlichungstag und Bekanntmachung des Hinweises auf die Patenterteilung: **30.10.2019**
(21) Anmeldenummer: 16788009.5
(22) Anmeldetag: 13.07.2016
(51) Int. Cl.: A61F 13/02, A61F 13/00, A61F 13/12, A61F 17/00

(54) **MEDIZINISCHES PFLASTER**
MEDICAL PLASTER
PANSEMENT MEDICAL

(30) Priorität: 15.09.2015 DE 202015006520 U; 03.11.2015 DE 102015118780
(43) Veröffentlichungstag der Anmeldung: 15.08.2018
(73) Patentinhaber: Stavrou, Petros, 22453 Hamburg (DE); Piontek, Andre, 20259 Hamburg (DE)
(72) Erfinder: STAVROU, Petros, 22453 Hamburg (DE)
(74) Vertreter: RGTH
(86) Internationale Anmeldenummer: PCT/DE2016/100314
(87) Internationale Veröffentlichungsnummer: WO 2017/045663

(56) Entgegenhaltungen:
- EP-A2- 0 441 418
- WO-A1-2007/020980
- CA-A1- 2 193 537
- US-A1- 2002 019 602
- US-A1- 2005 100 588
- US-A1- 2009 324 713

## Beschreibung

### Gebiet der Erfindung

Die Erfindung betrifft ein medizinisches Pflaster, mit einer Schicht aus textilem Gewebe oder Kunststoff, einer Klebstoffschicht und einer saugfähigen Auflageschicht, welche an der Gewebe- oder Kunststoffschicht befestigt ist.

### Hintergrund der Erfindung

Pflaster der oben genannten Art werden beispielsweise als Wundschnellverband verwendet, um Wunden bzw. kleinere Verletzungen abzudecken und die Wundheilung zu beschleunigen. Die Pflaster können ein Antiseptikum oder andere pharmazeutisch wirksame Stoffe enthalten. Nachteilig daran ist, dass die Wirkstoffmenge vorgegeben und nicht individuell dosierbar ist. Außerdem können die Wirkstoffe durch die Haut resorbiert werden und unerwünschte Wirkungen auslösen.

Pflaster mit einer Öffnung, um die Wunde zu betrachten, sind auch bekannt, siehe z.B. CA 2 193 537 A1 oder US 2002/019602 A1. Die saugfähige Auflageschicht ist aber am abdeckenden Verschluss befestigt.

Weiterhin gibt es sogenannte Kälte- oder Kühlpflaster, die für entzündete Stellen ohne äußere Verletzungen angewandt werden, wie beispielsweise Verstauchungen, Prellungen, Zerrungen und dergleichen. Ein derartiges Pflaster ist beispielweise aus der Offenlegungsschrift DE 38 32 451 A1 oder z.B. US 2009/324713 A1 bekannt. Diese Pflaster enthalten Wirkstoffe wie Menthol, die die Beschwerden durch Kühlung lindern sollen. Tatsächlich binden die Wirkstoffe aber lediglich an den Kälte-Rezeptor TRP Melastatin 8, sodass subjektiv zwar ein kühlender Effekt wahrgenommen, die Haut- bzw. Körpertemperatur jedoch physikalisch nicht erniedrigt wird. Gerade die physikalische Absenkung der Temperatur hat jedoch eine entzündungshemmende und schmerzlindernde sowie eine abschwellende Wirkung bei Verletzungen oder chirurgischen Eingriffen.

### Zusammenfassung der Erfindung

Die vorliegende Erfindung wurde vor dem Hintergrund des vorstehend beschriebenen Stands der Technik entwickelt. Aufgabe der Erfindung ist es, ein medizinisches Pflaster mit einer Schicht aus textilem Gewebe oder Kunststoff, einer Klebstoffschicht und einer saugfähigen Auflageschicht, welche an der Gewebe- oder Kunststoffschicht befestigt ist, vorzuschlagen, durch das die oben genannten Nachteile vermieden werden.

Diese Aufgabe wird dadurch gelöst, dass die Gewebe- oder Kunststoffschicht im Bereich der Auflageschicht eine Öffnung aufweist, sodass die Auflageschicht im Bereich der Öffnung freiliegt und zugänglich ist. Deshalb kann ein Wirkstoff auf die saugfähige Auflageschicht gegeben werden, die ein Wirkstoffreservoir bildet. Der dadurch erzielte Vorteil besteht darin, dass sowohl die Art des Wirkstoffs als auch die Wirkstoffmenge nicht vorgegeben ist, sondern je nach Bedarf durch Zugabe gewählt werden kann. Dies erlaubt eine auf den Einzelfall bezogene, individualisierte Therapie. Ein weiterer Vorteil besteht darin, dass der Wirkstoff nachdosiert werden kann, ohne dass das Pflaster entfernt werden muss. Ein erheblicher, zusätzlicher Vorteil besteht darin, dass das Pflaster als physikalisch wirksames Kühlpflaster verwendet werden kann. Durch die Öffnung kann eine Flüssigkeit in der Auflageschicht verdampfen und so physikalisch Verdampfungskälte erzeugen. Hierdurch wird die Temperatur der entzündeten Stelle tatsächlich gesenkt und ein antiinflammatorischer Effekt erzeugt, den herkömmliche Mentholpflaster nicht aufweisen. Das erfindungsgemäße Pflaster ist vielfältig einsetzbar, z.B. bei Arthritiden, Hämatomen, Schwellungen, Insektenstichen usw. Die Anwendung ist nicht auf den Menschen beschränkt, sondern das Pflaster kann auch in der Veterinärmedizin eingesetzt werden. Durch den Begriff "Pflaster" soll die Größe und Form des Pflasters nicht beschränkt werden, vielmehr kann das erfindungsgemäße Pflaster jede benötigte Größe und Form aufweisen. Die Auflageschicht ist durch die Klebstoffschicht mit der Schicht aus textilem Gewebe oder Kunststoff verbunden. Die Klebstoffschicht kann, muss aber nicht über den Bereich der Auflageschicht hinausgehen, sodass das Pflaster auf die Haut geklebt werden kann. Die Auflageschicht kann beispielsweise ein Gaze- oder Vliesstoff sein.

Vorteilhafte Ausgestaltungen der Erfindung mit zusätzlichen Merkmalen werden nachfolgend beschrieben.

Die Auflageschicht des Pflasters kann mindestens einen Wirkstoff aufweisen. Der Begriff "Wirkstoff" umfasst dabei physikalisch wirksame und/oder pharmazeutisch wirksame Substanzen. Beispielsweise kann der Wirkstoff im einfachsten Fall Wasser oder ein Alkohol oder ein Gemisch sein, das verdampft und so physikalisch Kälte erzeugt. Es kann sich auch um einen Wirkstoff handeln, der Wärme erzeugt und/oder abgibt. Der Wirkstoff kann auch ein Antiseptikum sein oder ein schmerzstillendes Mittel, ein Antibiotikum oder dergleichen. Die Wirkung von pharmazeutischen Stoffen kann topisch oder transdermal erfolgen. Möglich ist auch eine Kombination von unterschiedlichen Wirkstoffen.
In einer besonderen Ausführungsform als Kühlpflaster weist die Auflageschicht eine wässrige Menthollösung auf. Hierdurch wird ein kombinierter Effekt sowohl durch die Rezeptorbindung des Menthols als auch durch die physikalische Erniedrigung der Temperatur erzielt, welche durch eine Verdampfung der Menthollösung bewirkt wird.

Für die Öffnung kann ein abdeckender Verschluss vorgesehen sein, der vorzugsweise Poren aufweist, welche für gasförmige und/oder flüchtige Stoffe durchlässig sind. Hierdurch kann die Auflageschicht vor Verschmutzung geschützt werden und das Pflaster dennoch alle oben genannten Vorteile und Wirkungen aufweisen.

Die Erfindung umfasst außerdem eine Haube für den Kopf, die etwa in der Art einer Operationshaube ausgebildet sein kann. Diese weist an der Oberseite ein integrales medizinisches Pflaster mit oben beschriebenen Merkmalen auf. Die Haube kann beispielsweise bei Verletzungen oder nach operativen Eingriffen verwendet werden, etwa nach einer Haartransplantation. Die transplantierten Hautareale können dann gekühlt und/oder medikamentös versorgt werden.

Die Erfindung umfasst weiterhin ein medizinisches Set, welches ein medizinisches Pflaster mit oben beschriebenen Merkmalen oder die oben genannte Haube oder beide Teile umfasst. Zusätzlich ist ein Behälter mit mindestens einem Wirkstoff vorgesehen, um den Wirkstoff oder die Wirkstoffe auf das Pflaster oder die Haube mit dem Pflaster zu geben.

Das medizinische Set kann insbesondere einen Applikator zum Aufgeben des Wirkstoffes auf das Pflaster aufweisen oder der Behälter kann als Applikator ausgebildet sein.

Der Applikator kann als eine Pipette zum Aufträufeln des Wirkstoffes auf das Pflaster ausgebildet sein.

Vorzugsweise kann der Wirkstoff im Behälter oder Applikator eine wässrige Menthollösung sein. Sobald die Menthollösung verdampft ist, kann sie mit der Pipette nachgegeben werden.

### Kurze Beschreibung der Zeichnungen

Die Figuren zeigen im Einzelnen:
- Figur 1: eine schematische Draufsicht auf ein Pflaster;
- Figur 2: ein medizinisches Set aus Pipette, Wirkstoff und dem Pflaster aus Figur 1 im vertikalen Schnitt; und
- Figur 3: eine perspektivische Ansicht einer Haube für den Kopf mit einem Pflaster aus Figur 1.

Funktionsmäßig gleiche Teile sind mit denselben Bezugszeichen versehen.

### Detaillierte Beschreibung von bevorzugten Ausführungsformen

Nachfolgend werden bevorzugte Ausführungsformen der Erfindung unter Bezugnahme auf die Zeichnungen detailliert beschrieben, wobei weitere vorteilhafte Merkmale den Figuren der Zeichnung zu entnehmen sind.

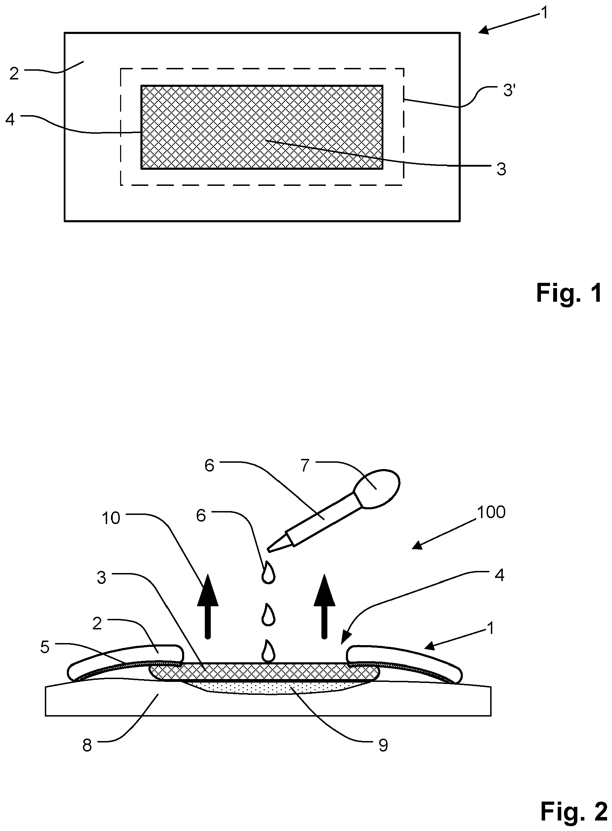

Figur 1 zeigt eine Draufsicht auf das Pflaster 1. Dieses weist eine obere Kunststoffschicht 2 auf. In der Kunststoffschicht 2 befindet sich eine Öffnung 4. Unterhalb der Kunststoffschicht 2 ist eine saugfähige Gazeschicht 3 angeordnet, die etwas größer als Öffnung 4 ist. Dieses ist durch die gestrichelten Linien 3' dargestellt. Die Gazeschicht 3 ist mit ihrem überlappenden Randbereich 3' an die Kunststoffschicht 2 geklebt.

Figur 2 zeigt ein medizinisches Set 100 aus Pipette 7, Wirkstoff 6 und dem Pflaster 1 aus Figur 1 im vertikalen Schnitt. Die Gazeschicht 3 ist mittels der Klebstoffschicht 5 an der oberen Kunststoffschicht 2 befestigt. In der Figur 2 ist außerdem ein Hautareal 8 mit einer entzündlichen Stelle 9 dargestellt. Das Pflaster 1 ist mit der unteren Klebstoffschicht 5 so auf das Hautareal 8 geklebt, dass die entzündliche Stelle 9 von der saugfähigen Gazeschicht 3 bedeckt ist.

Da innerhalb der der oberen Kunststoffschicht 2 eine Öffnung 4 vorgesehen ist, ist die Gazeschicht 3 von der Oberseite her zugänglich. Durch die Öffnung 4 kann mittels der Pipette 7 eine wässrige Menthollösung 6 auf die saugfähige Gazeschicht 3 geträufelt werden. Die Menthollösung 6 kann durch die Öffnung 4 verdampfen (als Pfeil 10 dargestellt) und entzieht so der entzündlichen Stelle 9 Wärme. Dies senkt die Hauttemperatur und wirkt lindernd, abschwellend und entzündungshemmend. Der Effekt wirkt durch das Menthol noch verstärkt.

Falls die wässrige Menthollösung 6 verdunstet ist, kann sie nachpipettiert werden, ohne dass das Pflaster 1 gewechselt werden muss.

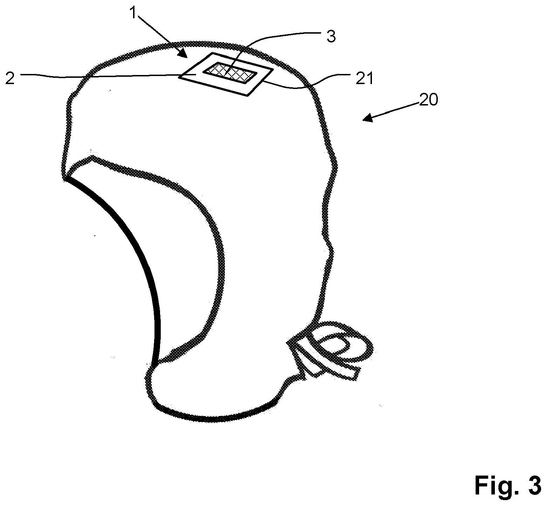

Figur 3 zeigt eine perspektivische Ansicht einer Haube 20 für den Kopf mit integriertem Pflaster 1. Die Haube 20 kann etwa eine übliche Operationshaube sein, die jedoch an einer dafür vorgesehenen Stelle eine Öffnung 21 aufweist. In der Figur 3 ist die Öffnung 21 an der Oberseite der Haube 20 angeordnet. Über dieser Öffnung 21 ist ein medizinisches Pflaster 1, wie es in den Figuren 1 und 2 beschrieben ist, angeordnet. Die Öffnung 21 ist etwas kleiner als das Pflaster 1, sodass dieses an den nicht gezeigten Rändern der Öffnung 21 durch Klebung befestigt ist.

Die Haube 20 kann beispielsweise bei Verletzungen oder auch nach operativen Eingriffen verwendet werden, etwa nach einer Haartransplantation. Die transplantierten Hautareale können dann gekühlt und/oder medikamentös versorgt werden.

### Bezugszeichenliste

- 1.: Pflaster
- 2.: Kunststoffschicht
- 3.: Gaze
- 4.: Öffnung
- 5.: Klebstoffschicht
- 6.: Wirkstofflösung
- 7.: Pipette
- 8.: Haut
- 9.: Entzündung
- 10.: Verdunstung
- 20.: Haube
- 21.: Öffnung
- 100.: Medizinisches Set

## Patentansprüche

1. Medizinisches Pflaster (1), mit einer Schicht aus textilem Gewebe oder Kunststoff (2), einer Klebstoffschicht (5) und einer saugfähigen Auflageschicht (3), welche an der Gewebe- oder Kunststoffschicht (2) befestigt ist, **dadurch gekennzeichnet, dass** die Gewebe- oder Kunststoffschicht (2) im Bereich der Auflageschicht (3) eine Öffnung (4) aufweist, sodass die Auflageschicht (3) im Bereich der Öffnung (4) freiliegt und zugänglich ist.

2. Medizinisches Pflaster (1) nach Anspruch 1, **dadurch gekennzeichnet, dass** die Auflageschicht (3) mindestens einen Wirkstoff (6) aufweist, insbesondere eine wässrige Menthollösung.

3. Pflaster (1) nach Anspruch 1 oder Anspruch 2, **dadurch gekennzeichnet, dass** für die Öffnung (4) ein abdeckender Verschluss vorgesehen ist, der vorzugsweise Poren aufweist, welche für gasförmige und/oder flüchtige Stoffe durchlässig sind.

4. Haube (20) für den Kopf, **dadurch gekennzeichnet, dass** sie ein Pflaster (1) nach einem der vorhergehenden Ansprüche aufweist.

5. Medizinisches Set (100), **dadurch gekennzeichnet, dass** es ein medizinisches Pflaster (1) und/oder eine Haube (20) nach einem der vorhergehenden Ansprüche sowie einen Behälter (7) mit mindestens einem Wirkstoff (6) umfasst.

6. Medizinisches Set nach dem vorhergehenden Anspruch, **dadurch gekennzeichnet, dass** es einen Applikator (7) zum Aufgeben des Wirkstoffes (6) auf das Pflaster (1) aufweist oder der Behälter als Applikator (7) ausgebildet ist.

7. Medizinisches Set nach dem vorhergehenden Anspruch, **dadurch gekennzeichnet, dass** der Applikator als Pipette (7) zum Aufträufeln des Wirkstoffes (6) auf das Pflaster ausgebildet ist.

8. Medizinisches Set (100) nach einem der Ansprüche 4 bis 6, **dadurch gekennzeichnet, dass** der Wirkstoff im Behälter oder Applikator (eine wässrige Menthollösung ist.

## Claims

1. Medical plaster (1) with a layer of textile fabric or plastic (2), an adhesive layer (5) and an absorbent dressing layer (3) which is attached to the layer of fabric or plastic (2), **characterized in that** the layer of fabric or plastic (2) has an opening (4) in the area of the dressing layer (3), so that the dressing layer (3) is exposed and accessible in the area of the opening (4).

2. Medical plaster (1) according to claim 1, **characterized in that** the dressing layer (3) has at least one active ingredient (6), in particular an aqueous menthol solution.

3. Plaster (1) according to claim 1 or claim 2, **characterized in that** a covering closure is provided for the opening (4), which preferably has pores which are permeable to gaseous and / or volatile substances.

4. Hood (20) for the head, **characterized in that** it has a plaster (1) according to one of the preceding claims.

5. Medical kit (100), **characterized in that** it comprises a medical plaster (1) and / or a hood (20) according to one of the preceding claims and a container (7) with at least one active ingredient (6).

6. Medical kit according to the preceding claim, **characterized in that** it has an applicator (7) for applying the active ingredient (6) to the plaster (1) or the container is designed as an applicator (7).

7. Medical kit according to the preceding claim, **characterized in that** the applicator is designed as a pipette (7) for applying the active ingredient (6) dropwise to the plaster.

8. Medical kit (100) according to any one of claims 4 to 6, **characterized in that** the active ingredient in the container or applicator is an aqueous menthol solution.

## Revendications

1. Pansement médical (1) avec une couche en tissu textile ou substance plastique (2), une couche d'adhésif (5) et une couche de support absorbante (3), laquelle est fixée à la couche de tissus ou de substance plastique (2), **caractérisé en ce que** la couche de tissu ou de substance plastique (2) comporte dans la zone de la couche de support (3) une ouverture (4) de sorte que la couche de support (3) est libérée et accessible dans la zone de l'ouverture (4).

2. Pansement médical (1) selon la revendication 1, **caractérisé en ce que** la couche de support (3) comporte au moins une substance active (6), notamment une solution mentholée aqueuse.

3. Pansement (1) selon la revendication 1 ou la revendication 2, **caractérisé en ce que** pour l'ouverture (4) une fermeture couvrante est prévue, qui comporte de préférence des pores, lesquels sont perméables aux substances gazeuses et/ou volatiles.

4. Bonnet (20) pour la tête, **caractérisé en ce qu'**il comporte un pansement (1) selon l'une quelconque des revendications précédentes.

5. Ensemble médical (100), **caractérisé en ce qu'**il comprend un pansement médical (1) et/ou un bonnet (20) selon l'une quelconque des revendications précédentes ainsi qu'un conteneur (7) avec au moins une substance active (6).

6. Ensemble médical selon la revendication précédente, **caractérisé en ce qu'**il comporte un applicateur (7) pour administrer la substance active (6) sur le pansement (1) ou le conteneur est constitué comme applicateur (7).

7. Ensemble médical selon la revendication précédente, **caractérisé en ce que** l'applicateur est constitué comme une pipette (7) pour verser goutte à goutte la substance active (6) sur le pansement.

8. Ensemble médical (100) selon l'une quelconque des revendications 4 à 6, **caractérisé en ce que** la substance active dans le conteneur ou l'applicateur est une solution mentholée aqueuse.
